# EUROPEAN PATENT APPLICATION

(11) **EP 2 127 662 A1**
(43) Date of publication of application: **02.12.2009**
(21) Application number: 08721043.1
(22) Date of filing: 29.02.2008
(51) Int. Cl.: A61K 38/00, A23K 1/16, A23L 1/30, A61P 19/00, A61P 19/02, A61P 19/10, A61P 29/00, A61P 37/02, A61P 37/08, A61P 43/00

(54) **RANKL PRODUCTION INHIBITOR**

(30) Priority: 02.03.2007 JP 2007052402
(71) Applicant: Snow Brand Milk Products Co., Ltd., Sapporo-shi Hokkaido 065-0043 (JP)
(72) Inventor: ONO, Aiko, Kawagoe-shi Saitama 350-1165 (JP); SERIZAWA, Atsushi, Kawagoe-shi Saitama 350-1165 (JP); MORITA, Yoshikazu, Kawagoe-shi Saitama 350-1165 (JP); KAWAKAMI, Hiroshi, Kawagoe-shi Saitama 350-1142 (JP)
(74) Representative: Wakerley, Helen Rachael
(86) International application number: PCT/JP2008/053595
(87) International publication number: WO 2008/108284

(57) **Abstract**

The production of RANKL may be inhibited by orally ingesting a milk-derived basic protein. Thus, a RANKL production inhibitor containing a milk-derived basic protein fraction as an active ingredient; a therapeutic agent for a metabolic bone disease and a therapeutic agent for an immune disease each containing the RANKL production inhibitor; and a food/beverage and a feed each containing a specified amount of the RANKL production inhibitor and being **characterized by** inhibiting the production of RANKL have a remarkable effect of inhibiting the production of RANKL, can be ingested on a daily basis, and exhibit high safety even when being ingested over a long period.

## Description

### Technical Field

The present invention relates to a Receptor Activator NF-κB Ligand (hereinafter, referred to as RANKL) production inhibitor containing a milk-derived basic protein fraction as an active ingredient, and a therapeutic agent for a metabolic bone disease and a therapeutic agent for an immune disease each containing the RANKL production inhibitor. In addition, the present invention relates to a food/beverage and a feed each containing a specified amount or more of the RANKL production inhibitor and being characterized by inhibiting the production of RANKL. The production of RANKL, which has not been effectively controlled by conventional methods, may be inhibited by orally ingesting the milk-derived basic protein fraction.

### Background Art

The bone is known as a dynamic organ which constantly remodels itself by repeating bone formation and bone resorption in order to change the own morphology and maintain a blood calcium concentration. In the normal bone, homeostasis is maintained by bone formation induced by osteoblasts and bone resorption induced by osteoclasts. However, the disruption of the balance between the bone formation and bone resorption leads to an abnormal bone metabolism as osteoporosis. As bone metabolism regulatory factors, there are exemplified a hormone, which is a systemic factor, and a cytokine, which is a topical factor, and those regulatory factors are responsible for the formation and maintenance of the bone.

RANKL (also referred to as OPGL, TRANCE or ODF) is a molecule which is been identified as a TNF f ami ly being expressed on osteoblasts, fibroblasts and the like and regulating osteoclast differentiation (see Non-patent Document 1) and which promotes osteoclast differentiation by binding to RANK which is a receptor being expressed on the osteoclast precursor cell membrane. Further, it is suggested that bone metabolism is closely associated with an immune cell function, based on the following facts: RANKL is a molecule also being expressed on activated T cells, and even RANKL produced by T cells regulates bone metabolism (see Non-patent Document 2). A dendritic cell (hereinafter, referred to as DC) is the most powerful antigen-presenting cell (APC), and in vivo, an antigen-presentation to T cells is required for starting a series of immune responses. DCs are also known to express RANK, which is a receptor, on the cell membrane, and it is said that the interaction between RANKL on the T cell membrane and RANK on the DC membrane is involved in an immune response (see Non-patent Documents 3 and 4). It is also said that soluble RANKL released from the T cell membrane regulates the maturation and activation of DCs (see Non-patent Documents 5 to 9).

For this reason, RANKL produced by T cells not only controls bone metabolism by regulating osteoclast differentiation via RANK, but also controls the immune system by being involved in the interaction between T cells and DCs and the maturation of DCs. Therefore, medicaments such as a RANKL production inhibitor may be expected to be used for the treatment and amelioration of a disease mediatedbyaRANKL-RANKsignal, for example, a metabolic bone disease such as osteoporosis, and an immune disease such as rheumatoid arthritis and allergic disease due to the abnormal activation of the immune response.

The intestinal tract is an organ to which a food directly contacts. The intestinal tract not only absorbs nourishment simply, but also receives various signals from a food and transmits various signals through a nerve and a hormone to the whole body. In addition, as a front line of biophylaxis, in order to face off against a number of foreign substances (a food, an allergen, an indigenous bacterium in the mucosa, and a carcinogen) including a microbial pathogen, there is arranged the maximum immune system in the human body, which contributes to the maintenance of the homeostasis of the living body. Therefore, it may be expected to be highly effective for an immune disease if a food ingredient which acts on the immune system can be ingested from a diet to be ingested on a daily basis. Nevertheless, in general, pharmaceutical products are used for the treatment of a bone disease and an immune disease. However, those substances are pharmaceuticals themselves, and one can never say that they are highly safe. Thus, in view of the problems of the proper amount and the like, it is difficult to comfortably take those substances from a daily diet. So, if it is possible to control RANKL acting on the immune system of the intestinal tract and being produced by T cells which are immunocompetent cells, the inhibition of bone resorption may be expected by inhibiting the differentiation and maturation of osteoclasts in the bone. The inhibition of bone resorption leads to bone reinforcement. Similarly, the inhibition of an immune disease due to an abnormal immune reaction such as an allergy may also be expected.

Accordingly, in a comparison with the ingestion of the RANKL production inhibitor as the drug described above, if there is realized the ingestion of a food ingredient acting on the immune system and being obtained from a substance which may be ingested on a daily basis, is free of problems even though ingestion over a long period, and may also be used as a food material, it may be expected that the food ingredient is highly effective for an immune disease. Thus, it is strongly desired to develop such an inhibitor.
Now, there has not been reported any ingredient which has an action of inhibiting the production of RANKL in T cells and which may also be used as a food material, and hence, the present invention is a novel technology.

On the other hand, a milk-derived basic protein fraction collectively refers to multiple basic proteins contained in milk in a trace amount, and is extracted as a basic protein fraction from a milk raw material such as skim milk and whey. Then, the milk-derived basic protein fraction is known to have a bone reinforcing action through oral ingestion (see Patent Document 1). However, with regard to the bone reinforcing action of the milk-derived basic protein fraction, it is known that the milk-derived basic protein fraction inhibits the differentiation and maturation by directly acting on osteoclasts, but it is not known that the milk-derived basic protein fraction inhibits the production of RANKL in cells. The inventors of the present invention have focused on RANKL which is said to be involved in bone metabolism, and have investigated a food ingredient inhibiting the production of RANKL in intestinal tract immune cells to which a food ingredient directly contacts.
[Non-patent Document 1] Suda et al., Endcr. Rev., 20: 345 (1999)
[Non-patent Document 2] Theil et al., Annu. Rev. Immunol., 20: 795 (2002)
[Non-patent Document 3] Hochweller et al., Eur. J. Immunol., 35: 1086 (2005)
[Non-patent Document 4] Williamson et al., J Immunol., 169: 3606 (2002)
[Non-patent Document 5] Wong et al., J. Exp. Med. , 186: 2075 (1997)
[Non-patent Document 6] Wong et al., J. Leukocyte Biol., 65: 715 (1999)
[Non-patent Document 7] Wong et al., J. Biol. Chem., 272: 25190-25194 (1997)
[Non-patent Document 8] Josien et al. , J. Immunol., 162: 2562 (1999)
[Non-patent Document 9] Josien et al., J. Exp. Med. , 191: 495 (2000)
[Patent Document 1] JP-A-H08-151331

### Disclosure of the Invention

### Problems to be solved by the Invention

It is an object of the present invention to provide: a RANKL production inhibitor containing a milk-derived basic protein fraction as an active ingredient, which may be ingested on a daily basis and exhibits high safety even though ingestion over a long period; and a therapeutic agent for a metabolic bone disease and a therapeutic agent for an immune disease each containing the RANKL production inhibitor, each of which has an action of inhibiting osteoclast differentiation or an action of inhibiting dendritic cell differentiation through the inhibition of the production of RANKL, to thereby be able to treat a bone disease or an immune disease and an allergy. It is another object of the present invention to provide a food/beverage and a feed each containing a specified amount of the RANKL production inhibitor and being characterized by inhibiting the production of RANKL.

### Means for solving the Problems

The inventors of the present invention have continuously investigated a substance having an action of inhibiting the production of RANKL for the purpose of obtaining a substance which inhibits osteoclast differentiation or dendritic cell differentiation through the inhibition of the production of RANKL. As a result, the inventors have discovered that a basic protein which is present in milk only in a trace amount inhibits the production of RANKL on the T cell membrane and soluble RANKL. Then, the inventors have found that the milk-derived basic protein fraction may be utilized as an active ingredient of the RANKL production inhibitor, and thus have completed the present invention. That is, the present invention is a RANKL production inhibitor containing a milk-derived basic protein fraction as an active ingredient. The present invention further relates to a therapeutic agent for a metabolic bone disease and a therapeutic agent for an immune disease each containing the RANKL production inhibitor. The present invention still further relates to a food/beverage and a feed each containing a specified amount of the RANKL production inhibitor and being characterized by inhibiting the production of RANKL.

### Effects of the Invention

The production of RANKL may be inhibited by using the RANKL production inhibitor containing a milk-derived basic protein fraction as an active ingredient of the present invention. The RANKL production inhibitor, the therapeutic agent for a metabolic bone disease and the therapeutic agent for an immune disease each containing the RANKL production inhibitor, and the food/beverage or the feed containing the RANKL production inhibitor and being characterized by inhibiting the production of RANKL, of the present invention may be used for the treatment, symptom amelioration, or the like of a metabolic bone disease through the inhibition of osteoclast differentiation, or may be used for the treatment, symptom amelioration, or the like of a disease involved in an immune function such as an allergic disease through the inhibition of dendritic cell differentiation to regulate an immune function, and hence are very useful.

### Brief Description of the Drawings

FIG. 1 is a graph illustrating the effect of a milk-derived basic protein fraction for a production amount of RANKL, which is determined by an antigen-nonspecific TCR stimulation method (Example 1 and Test Example 1).
FIG. 2 is a graph illustrating the effect of the milk-derived basic protein fraction for a production amount of RANKL, which is determined by an antigen-specific TCR stimulation method (Example 1 and Test Example 2).

### Best Mode for carrying out the Invention

A RANKL production inhibitor of the present invention is characterized by containing a milk-derived basic protein fraction as an active ingredient. Further, by compounding the RANKL production inhibitor containing a milk-derived basic protein fraction as an active ingredient to inhibit the production of RANKL and inhibit osteoclast differentiation, the RANKL production inhibitor is utilized for the treatment of a metabolic bone disease. In addition, by inhibiting the production of RANKL and inhibiting dendritic cell differentiation to regulate an immune function, the RANKL production inhibitor is utilized for the treatment of a disease involved in an immune function.

In the RANKL production inhibitor, the therapeutic agent for a metabolic bone disease and the therapeutic agent for an immune disease each containing the RANKL production inhibitor, and the food/beverage and the feed containing a specified amount of the RANKL production inhibitor and being characterized by inhibiting the production of RANKL, of the present invention, a milk-derived basic protein fraction obtained from mammalian milk such as cow milk, human milk, goat milk, and sheep milk as a raw material can be used, as an active ingredient.

As a method of obtaining the milk-derived basic protein fraction which is an active ingredient of the RANKL production inhibitor of the present invention, there are known for example: a method of obtaining the fraction by bringing milk or a milk-derived raw material into contact with a cation exchanger to adsorb a milk-derived basic protein fraction, and then eluting the basic protein fraction adsorbed to the cation exchanger with an eluent having a pH of more than 5 and an ionic strength of more than 0.5 (JP-A-H05-202098); a method of obtaining the fraction by using an alginic acid gel (JP-A-S61-246198); a method of obtaining the fraction from whey by using inorganic porous particles (JP-A-H01-86839); and a method of obtaining the fraction from milk byusingasulfatedestercompound (JP-A-S63-255300). In the present invention, there may be used a milk-derived basic protein fraction obtained by such methods.

In order to obtain an effect of inhibiting the production of RANKL, as effective amounts of the RANKL production inhibitor and the food/beverage containing a specified amount of the RANKL production inhibitor and being characterized by inhibiting the production of RANKL, of the present invention, 20 mg/day or more of the milk-derived basic protein fraction based on the solid content are desirably ingested by an adult human. Then, in the RANKL production inhibitor or the food/beverage containing the RANKL production inhibitor, the milk-derived basic protein fraction is desirably compounded in an amount of 10 mg to 100 g/100 g based on the solid content.

In the RANKL production inhibitor of the present invention, the milk-derived basic protein fraction may be used alone or in combination with other ingredients mentioned below. There may be formulated into a shape such as powder, liquid, or a tablet depending on purpose of use and method of use, for example.

The RANKL production inhibitor of the present invention may also be constructed in a form of a nutrient composition by using a protein, a carbohydrate, a lipid, vitamins, minerals or the like mentioned below as a main ingredient. The nutrient composition having an effect of inhibiting the production of RANKL is also processed into a shape such as powder, liquid, or a tablet depending on purpose of use and method of use, for example.

Further, the RANKL production inhibitor of the present invention may be added to a food/beverage mentioned below, and then processed by a conventional method to obtain also the food/beverage containing the RANKL production inhibitor and being characterized by inhibiting the production of RANKL.
In preparing the RANKL production inhibitor or the food/beverage containing the RANKL production inhibitor and being characterized by inhibiting the production of RANKL, of the present invention, examples of the protein may include: a milk protein fraction such as casein, whey protein concentrate (WPC), whey protein isolate (WPI), αs-casein, β-casein, α-lactalbumin and β-lactoglobulin; and a vegetable protein such as a soybean protein and a wheat protein; and the like. In addition, those proteins may be used in a form of a peptide or a free amino acid by treating with an acid or an enzyme. The free amino acid may be used not only as a nitrogen source, but also for imparting a specif iedphysiological action. Examples of these amino acids may include taurine, cystine, cysteine, arginine, glutamine and the like. Those proteins and peptides or free amino acids are preferably compounded in an amount of 5 to 30% by weight based on the solid component of the RANKL production inhibitor or the food/beverage containing the RANKL production inhibitor and being characterized by inhibiting the production of RANKL.

Examples of the carbohydrate may include starch, soluble polysaccharides, dextrin, sucrose, lactose, maltose and glucose, an oligosaccharide such as galactosyl lactose, a fructo oligosaccharide and lactulose, an artificial sweetener, or the like. The carbohydrate is preferably compounded in an amount of 40 to 80% by weight based on the solid component of the RANKL production inhibitor or the food/beverage containing the RANKL production inhibitor and being characterized by inhibiting the production of RANKL.

Examples of the lipid may include: animal oil and fat such as milk fat, lard, beef fat and fish oil; a vegetable oil such as soybean oil, rapeseed oil, corn oil, evening primrose oil, medium-chain triglyceride (MCT) and cottonseed oil; and further, a separated oil, hydrogenated oil and ester-exchanged oil thereof. The lipid is preferably compounded in an amount of 40% by weight or less based on the solid component of the RANKL production inhibitor or the food/beverage containing the RANKL production inhibitor and being characterized by inhibiting the production of RANKL.

As the vitamins and minerals, for example, vitamins and minerals in designated additives according to the Food Sanitation Law (food additives listed in Annexed Table 2 of the Enforcement Regulations) and existing food additives (food additives listed in the list of existing additives under the Food Sanitation Law) may be used. Specific examples of the vitamins may include vitamin A, vitamin B's, vitamin C, vitamin D, vitamin E, vitamin K's, folic acid, pantothenic acid, β-carotene, a nicotinamide, biotin, inositol, choline and the like. The vitamins are preferably compounded in an amount of 0.01 to 5% by weight based on the solid component of the RANKL production inhibitor or the food/beverage containing the RANKL production inhibitor and being characterized by inhibiting the production of RANKL. Further, specific examples of the minerals may include calcium, magnesium, potassium, sodium, phosphorus, chlorine, iron, copper, zinc, iodine, manganese, selenium, fluorine, chromium, molybdenum and the like. The minerals are preferably compounded in an amount of 0.001 to 5% by weight based on the solid component of the RANKL production inhibitor or the food/beverage containing the RANKL production inhibitor and being characterized by inhibiting the production of RANKL.

In addition, examples of the food/beverage containing the RANKL production inhibitor of the present invention include: a milk food such as cheese, butter, and fermented milk; a beverage such as a milk beverage, drinking yogurt, a coffee beverage, and fruit juice; a confectionery such as a jelly, a pudding, a cookie, a biscuit, and a wafer; and various foods/beverages such as modified milk for infant rearing, follow-up milk, a food/beverage for infants, a food/beverage for pregnant women, a food for disease conditions, a medical food, a food for the elderly, a nursing care food, and further, a frozen food. In manufacturing the food/beverage containing the RANKL production inhibitor, the milk-derived basic protein fraction is relatively unstable against heat, and hence, particularly in a heat sterilization step, it is desired to keep the thermal history as low as possible.

Next, the present invention is described in detail by way of Examples and Test Examples. Those examples merely illustrate embodiments of the present invention, and the present invention is in no way limited thereto.

### [Example 1]

### (Preparation of milk-derived basic protein fraction)

A column (diameter 5 cm × height 30 cm) filled with 400 g of a cation-exchange resin, sulfonated Chitopal (manufactured by Fuji Spinning Co., Ltd.) was sufficiently washed with deionized water. Then, 40 1 of unsterilized skim milk (pH 6.7) were passed through the column at a flow rate of 25 ml/min, after which the column was sufficiently washed with deionized water, and subsequently washed with a 0.02 M carbonate buffer (pH 7.0) containing 0.7 M sodium chloride. After that, the milk-derived basic protein fraction adsorbed to the resin was eluted with a 0.02 M carbonate buffer (pH 7.0) containing 0.98 M sodium chloride. Then, the eluate was desalted using a reverse osmosis (RO) membrane and concentrated, followed by lyophilization, to thereby obtain 21 g of milk-derived basic protein fraction powder as the RANKL production inhibitor of the present invention.

### [Test Example 1]

### (The effect of milk-derived basic protein fraction for production amount of RANKL determined by antigen-nonspecific TCR stimulation method)

From the lymph nodes of 6 to 8-week-old BALB/C male mice, CD4-positive cells were fractionated by a Macs (magnetic cell separation system) method. The cells were suspended into an RPMI 1640 medium (manufactured by Nipro Corporation, containing 5% fetal bovine serum, further containing a 1% penicillin-streptomycin solution (manufactured by GIBCO); hereinafter, referred to as 5% FCS/RPMI) so as to achieve a concentration of 4×10⁶ cells/ml, and then seeded onto an anti-mouse CD3 antibody-coated 96-well plate (manufactured by Becton, Dickinson and Company) in a volume of 50 µl/well (2×10⁵ cells/well). An anti-mouse CD28 antibody was added so as to achieve a final concentration of 2.5 µg/ml, and further, the milk-derived basic protein fraction obtained in Example 1 or bovine serum albumin (hereinafter, referred to as BSA) as a control were each added so as to achieve final concentrations of 0, 10, and 100 µg/ml, followed by cultivation in a 5% CO₂ incubator. After 4 days, the supernatant was collected, and the amount of RANKL in the supernatant was measured by an ELISA method.

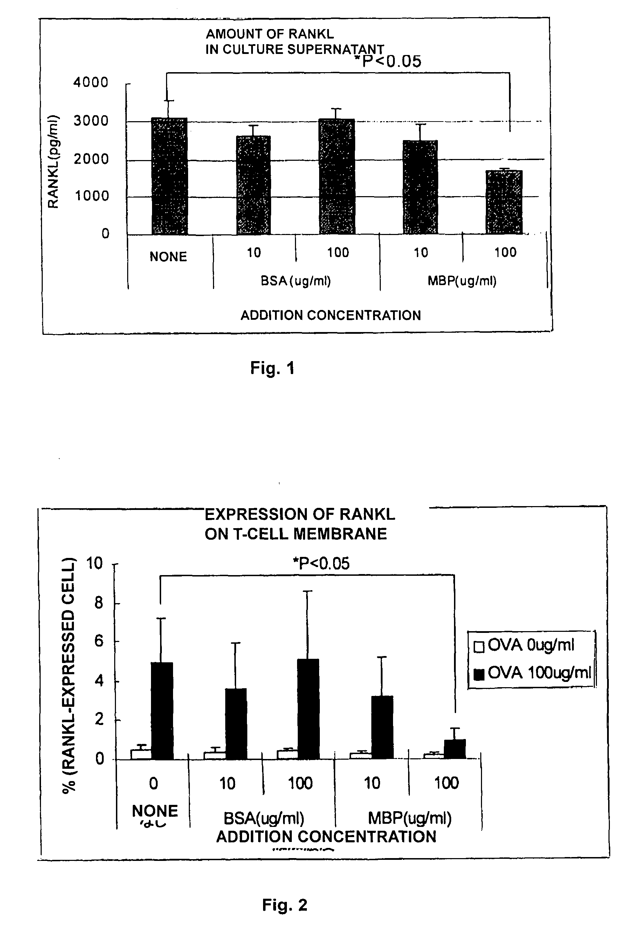

The results of the amount of RANKL were shown in Fig. 1. When T cells were cultivated under a TCR stimulation condition with an antibody, the amount of RANKL in the culture supernatant was increased. On this occasion, the addition of BSA did not affect the production amount of RANKL, but in the cultivation with the addition of the milk-derived basic protein fraction the production of RANKL was inhibited. The results indicated that the milk-derived basic protein fraction directly inhibited the production of RANKL in T cells under an antigen-nonspecific TCR stimulation condition. The results indicate the possibility that in a metabolic bone disease in which the balance between bone formation and bone resorption is morbidly inclined to bone resorption, the milk-derived basic protein fraction inhibits bone resorption through the inhibition of osteoclast differentiation by an action of blocking a RANKL-RANK signal transmission between T cells and osteoclasts. It may also be expected that the milk-derived basic protein fraction directly acts on osteoblasts or fibroblasts which produce RANKL and inhibits the production of RANKL, to thereby inhibit bone resorption.

### [Test Example 2]

### (Effect of milk-derived basic protein fraction for expression of RANKL determined by antigen-specific TCR stimulation method)

From the lymph nodes of ovalbumin-specific T cell antigen receptor-Itransgenic male mice (Do11.10 mice), CD4-positive T cells were fractionated by the Macs method. The cells were suspended into 5% FCS/RPMI so as to achieve a concentration of 4×10⁶ cells/ml, and then seeded onto a 48-well microplate in a volume of 100 µl/well (4×10⁵ cells/well). From the Peyer's patch cells of BALB/C male mice, CD11c-positive cells (dendritic cells) were fractionated by the Macs method, suspended into 5% FCS/RPMI so as to achieve a concentration of 4×10⁵ cells/ml, and then seeded in a volume of 100 µl/well (2×10⁴cells/well). Themilk-derivedbasicproteinfraction obtained in Example 1 and BSA as a control were each added so as to achieve final concentrations of 0, 10, and 100 µg/ml. In addition, ovalbumin (hereinafter, referred to as OVA) was added so as to achieve final concentrations of 0 and 100 µg/ml, followed by cultivation in a 5% CO₂ incubator. After 4 days, the respective cells were collected and stained with a PE-labeled anti-mouse RANKL antibody, an FITC-labeled anti-mouse KJ1.26 antibody which was a specific antibody to TCR of Do11.10 mice, and PI, and the ratio of RANKL-positive and KJ1.26-positive cells (%) were determined by a flow cytometry method.

The results were shown in Fig. 2. Even when the milk-derived basic protein fraction or BSA was added under a condition without a stimulation caused by OVA which was an antigen, the expression of RANKL was not observed. On the other hand, when cultivation was performed under OVA stimulation, the expression of RANKL was increased. It was confirmed that the addition of BSA did not affect the expression of RANKL, but the addition of the milk-derived basic protein fraction remarkably inhibited the expression of RANKL. As the results, it was indicated that under an antigen-specific TCR stimulation, the milk-derived basic protein fraction inhibited the expression of RANKL on T cells. The results indicate the possibility that in an immune disease exhibiting an abnormally activated antigen-antibody reaction, the milk-derived basic protein fraction ameliorates an allergy and an autoimmune disease by inhibiting an immune reaction through an action of interfering a RANKL-RANK signal between T cells and dendritic cells which is an initial reaction of an immune response.

### Example 2

By using the milk-derived basic protein powder obtained in Example 1, the RANKL production inhibitor in a form of powder having composition as shown in Table 1 was prepared by a conventional method. The RANKL production inhibitor contained 4 g of the milk-derived basic protein fraction per 100 g.

**[Table 1]**

| | |
|---|---|
| Hydrous crystalline glucose | 77.5 (Wt%) |
| Soybean protein | 12 |
| Mineral mixture | 5 |
| Sugar ester | 1 |
| Flavor | 0.5 |
| Milk-derived basic protein fraction powder (Example 1) | 4 |

### [Example 3]

### (Manufacture of RANKL production inhibitor-containing beverage)

As shown in Table 2, 40 g of the basic protein powder obtained in Example 1 were dissolved in 50 L of deionized water having a pH adjusted to 3.2 with lactic acid. After that, 1 kg of sugar and 100 g of flavor were dissolved, followed by heat sterilization at 90°C for 15 minutes. 50 ml each of the resultant were hermetically filled into a lidded glass bottle to manufacture a RANKL production inhibitor-containing beverage characterized by inhibiting the product ion of RANKL. The beverage contained 80 mg of the milk-derived basic protein fraction per 100 ml.

**[Table 2]**

| | |
|---|---|
| Milk-derived basic protein fraction powder (Example 1) | 40 (g) |
| Sugar | 1,000 |
| Flavor | 100 |
| Lactic acid | 1,000 |
| Deionized water | 49,000 |

### Example 4

### (Manufacture of RANKL production inhibitor-containing biscuit characterized by inhibiting production of RANKL)

A dough having composition as shown in Table 3 was prepared, formed, and then baked to manufacture a RANKL production inhibitor-containing biscuit characterized by inhibiting the production of RANKL. The biscuit contained 100 mg of the milk-derived basic protein fraction per 100 g.

**[Table 3]**

| | |
|---|---|
| Wheat flour | 40 (g) |
| Sugar | 10 |
| Salt | 0.5 |
| Margarine | 12.5 |
| Egg | 11.5 |
| Water | 5.5 |
| RANKL production inhibitor (Example 2) | 20 |

### Example 5

### (Manufacture of RANKL production inhibitor-containing feed characterized by inhibiting production of RANKL)

The milk-derived basic protein fraction powder prepared in Example 1 was used, and each ingredient was stirred in accordance with the formulation as shown in Table 4 to homogenize, and thereby preparing a RANKL production inhibitor-containing feed characterized by inhibiting the production of RANKL of the present invention. The feed contained 100 mg of the milk-derived basic protein fraction per 100 g.

**[Table 4]**

| | | |
|---|---|---|
| Casein | 19.9 | (wt%) |
| α-corn starch | 15.0 | |
| Cellulose | 5.0 | |
| Corn oil | 5.0 | |
| Vitamin mixture | 1.0 | |
| Mineral mixture (calcium-free) | 3.5 | |
| Sucrose | 48.91 | |
| Calcium hydrogen phosphate dodecahydrate | 1.29 | |
| DL-Methionine | 0.3 | |
| Milk-derived basic protein fraction powder (Example 1) | 0.1 | |

### Industrial Applicability

The RANKL production inhibitor containing a milk-derived basic protein fraction as an active ingredient, the therapeutic agent for a metabolic bone disease and the therapeutic agent for an immune disease each containing the RANKL production inhibitor, and further, the food/beverage or the feed containing the RANKL production inhibitor and being characterized by inhibiting the production of RANKL, of the present invention may be used for the treatment, symptom amelioration or the like of various diseases relating to a RANKL-RANK signal, and hence are very useful.

## Claims

1. A RANKL production inhibitor, comprising a milk-derived basic protein fraction as an active ingredient.

2. A therapeutic agent for a metabolic bone disease, comprising the RANKL production inhibitor according to claim 1.

3. A therapeutic agent for an immune disease, comprising the RANKL production inhibitor according to claim 1.

4. A food/beverage, comprising the RANKL production inhibitor according to claim 1 in an amount of 10 mg to 100 g/100 g based on a solid content.

5. A feed, comprising the RANKL production inhibitor according to claim 1 in an amount of 10 mg to 100 g/100 g based on of a solid content.
